Europäisches Patentamt

(19) **European Patent Office**

**Office européen des brevets**

(11) Veröffentlichungsnummer: **0 009 788**
**B1**

(12) **EUROPÄISCHE PATENTSCHRIFT**

(45) Veröffentlichungstag der Patentschrift:
28.07.82

(21) Anmeldenummer: **79103717.9**

(22) Anmeldetag: **01.10.79**

(51) Int. Cl.³: **C 07 C 121/16, C 07 C 120/00**

(54) Verfahren zur Herstellung von Monochlorazetonitril.

(30) Priorität: **11.10.78 DE 2844200**
**10.02.79 DE 2905082**

(43) Veröffentlichungstag der Anmeldung:
**16.04.80 Patentblatt 80/8**

(45) Bekanntmachung des Hinweises auf die Patenterteilung:
**28.07.82 Patentblatt 82/30**

(84) Benannte Vertragsstaaten:
**CH DE FR GB IT NL**

(56) Entgegenhaltungen:
**DE-B-1 065 405**
**GB-A-568 670**
**US-A-3 121 735**
**US-A-3 134 808**
**US-A-3 418 228**
**US-A-3 825 581**
**US-A-3 828 088**
**US-A-3 923 860**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Schubart, Rüdiger, Dr., An der Engelsfuhr 27, D-5060 Bergisch-Gladbach (DE)**
Erfinder: **Braden, Rudolf, Dr., Nothauser Feld 1, D-5068 Odenthal-Scheuren (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

Verfahren zur Herstellung von Monochloracetonitril

Die vorliegende Erfindung betrifft ein Verfahren zur selektiven Herstellung von Monochloracetonitril durch direkte Chlorierung von Acetonitril.

Es ist bekannt, dass bei direkter Einwirkung von Chlor auf Acetonitril in Gegenwart von Jod als Katalysator gemäss Ber.dtsch.chem. Ges. 9 1594 (1876) oder bei 435 bis 440 °C in der Dampfphase an einem Silber-haltigen Kontakt bzw. bei 65 °C in flüssiger Phase unter Bestrahlung durch UV-Licht gemäss Angaben in Houben-Weyl, Methoden der organischen Chemie, Bd. V/3, Seite 634, Georg Thieme Verlag Stuttgart (1962) praktisch ausschliesslich Trichloracetonitril entsteht. Auch die Anwendung der Bedingungen zur Herstellung von Monochlorisobutyronitril gemäss DE-PS 1 065 405 führt allein zur Perchlorierung des Acetonitrils. Relativ glatt verläuft hingegen die Monochlorierung eines stark aktivierten Acetonitrils, nämlich des Trichlorphenylacetonitrils (vgl. US-PS 3 134 808 ).

Zur Vermeidung der vollständigen Perhalogenierung von Acetonitril ist bereits vorgeschlagen worden, die Chlorierung bei relativ niedrigen Temperaturen nur bis zu einem bestimmten Chlorierungsgrad voranzutreiben und dann das Reaktionsgemisch aufzuarbeiten, wobei Ausbeuten an reinem Monochloracetonitril von etwa 40% erreicht werden können (vgl. US-PS 3 825 581 .

Deutlich bessere Ausbeuten erhält man erst durch Gasphasenchlorierung bei extrem hohen Temperaturen (maximal 380 °C) nach den Angaben in Bull. Soc. Chim. Belg. 61, 366 (1952) und Khum. Prom-st.' (Moskau) 53, 662 (1977).

Es wurde nun überraschend gefunden, dass man Monochloracetonitril in hoher Selektivität und bei niedrigen Temperaturen, nämlich bei Siedetemperatur des Acetonitrils, durch direkte Chlorierung in flüssiger Phase unter Einwirkung von UV-Strahlung und gegebenenfalls in Gegenwart von Halogeniden der 3.– 6. Gruppe des Periodensystems erhält, wenn man die Chlorierung kontinuierlich so weit durchführt, dass die Konzentration des entstandenen Monochloracetonitrils in dem kontinuierlich aus dem Reaktionsraum abgezogenen Reaktionsgemisch nicht mehr als etwa 33 Mol-%, vorzugsweise 2 bis 25 Mol-%, bezogen auf die Molzahl aller im Reaktionsgemisch enthaltenen Stoffe, beträgt.

Dabei sind die weiteren Reaktionsbedingungen, insbesondere der Chlorstrom und die Verweilzeit des Reaktionsgemisches im Reaktionsraum, so zu wählen, dass das Chlor praktisch vollständig umgesetzt wird.

Geeignete Halogenide von Elementen der 3. bis 6. Gruppe des Periodensystems sind beispielsweise Fluoride, Chloride, Bromide, Jodide, Oxychloride und Oxybromide, bevorzugt die Chloride und Bromide, von Bor, Aluminium, Gallium, Silicium, Titan, Germanium, Zinn, Blei, Zirkon, Phosphor, Arsen, Antimon, Vanadin, Niob, Tantal, Schwefel, Selen, Tellur, Chrom, Molybdän Wolfram, bevorzugt Gallium, Silicium, Titan, Germanium, Zinn, Phosphor, Arsen, Antimon und Schwefel.

Besonders bevorzugt sind solche Halogenide, die bei Reaktionstemperatur einen Dampfdruck von wenigstens 0,05 bar aufweisen und die sich in diesem Temperaturbereich nicht zersetzen. Als Beispiele hierfür seien genannt: Titantetrachlorid, Siliciumtetrachlorid, Schwefeldichlorid, Zinntetrachlorid, Gallium-III-Chlorid, Germaniumtetrabromid, Germaniumtetrachlorid, Phosphortribromid, Phosphortrichlorid, Arsentrichlorid, Arsentribromid und Antimonpentachlorid. Bevorzugt ist die Verwendung von Zinntetrachlorid. Das Halogenid wird in einer Menge von 0,1 bis 20 Gew.-%, bevorzugt 1 bis 8 Gew.-%, bezogen auf das eingesetzte Acetonitril, eingesetzt.

Der Reaktionsraum zur Durchführung des Verfahrens wird zweckmässigerweise so gestaltet, dass das Acetonitril und das Halogen gut vermischt werden. Beispielsweise kann der Reaktionsraum Einbauten, wie Umlenkbleche oder statische Mischer, haben oder als Venturi-Rohr gestaltet sein. Das Halogen kann auch über eine Fritte oder eine Ringdüse in das vorbeiströmende Acetonitril eingebracht werden.

Das Reaktionsgemisch kann durch übliche Massnahmen aufgearbeitet werden. Das bei der Aufarbeitung erhältliche Endprodukt kann anschliessend, beispielsweise durch fraktionierte Destillation, weiter gereinigt werden. Bei der Aufarbeitung können das im Überschuss eingesetzte Acetonitril und das oben beschriebene Halogenid zurückgewonnen werden und wieder in das erfindungsgemässe Verfahren eingesetzt werden.

Das nach dem erfindungsgemässen Verfahren herstellbare Monochloracetonitril wird in hohen Ausbeuten von über 90% der theoretischen Ausbeute, bezogen auf umgesetztes Acetonitril, und in besonders hoher Reinheit erhalten, so dass es für viele Anwendungen, beispielsweise zur Synthese von Tetrachlorpyrimidin, das ein wichtiges Ausgangsprodukt zur Herstellung von Reaktivfarbstoffen darstellt, ohne weitere Reinigung eingesetzt werden kann.

Durch die erfindungsgemässe Rückführung des nicht umgesetzten Acetonitrils, das vom Reaktionsgemisch nach Verlassen des Reaktionsraumes abgetrennt wird, können hohe Umsätze ohne Unterbrechung des Verfahrens erzielt werden.

Durch die erfindungsgemässe Rückführung des gegebenenfalls eingesetzten Halogenids eines Elements der 3. bis 6. Gruppe des Periodensystems ist ein besonders wirtschaftlicher Einsatz dieses Hilfsstoffs möglich.

**Beispiele**

A) **Reaktionsapparatur**

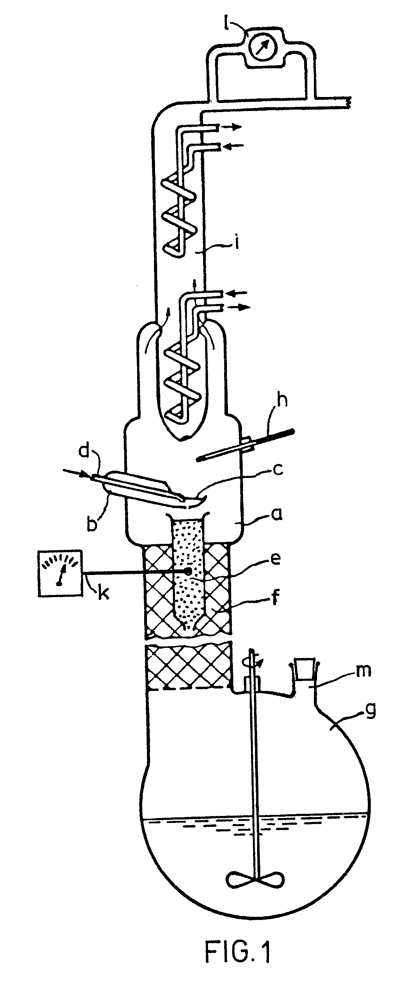

In den folgenden Beispielen wird die in Fig. 1 dargestellte Reaktionsapparatur verwendet. Die Apparatur besteht aus einem Verdampfungsgefäss (g), welcher das Acetonitril und das gegebenenfalls eingesetzte Halogenid enthält. Auf das Verdampfungsgefäss (g), ist eine mit Füllkörnern gefüllte Kolonne (f) aufgesetzt, in die ein leeres, sich nach unten verjüngendes Rohr (e) eingelassen ist. Oberhalb der Kolonne (f) befindet sich der Reaktionsraum (a), in den eine Vorrichtung (b) eingelassen ist, deren im Reaktionsraum befindlicher Teil zu einer Vertiefung (c) ausgebildet ist. Die Zuführung des Chlors erfolgt von aussen durch den Einlass (d) in die Vorrichtung (b) zu der Vertiefung (c), wobei gleichzeitig der Reaktionsraum mit UV-Licht bestrahlt wird. Zur Überprüfung der Reaktionstemperatur ist ausserdem in dem Reaktionsraum (a) das Thermometer (h) eingelassen.

Oberhalb des Reaktionsraumes ist der Kondensator (i) so angeordnet, dass die hier kondensierten flüssigen Komponenten in die Vertiefung (c) tropfen können.

In dem Rohr ist eine Probeentnahmevorrichtung (k) untergebracht, durch welche in beliebigen Abständen das unmittelbare Reaktionsgemisch einem IR-Spektrometer oder Gaschromatographen zugeführt werden kann. Am oberen Ende der Apparatur ist im by-pass ein gegebenenfalls akustisch arbeitendes Chlormessgerät (l) angebracht. Durch den am Verdampfungsgerät (g) befindlichen Hals (m) können Proben zur Analyse des in (m) angesammelten Produktes entnommen werden.

## B) Halogenierung

200 g Acetonitril und 5 g Zinntetrachlorid werden in der unter A) beschriebenen Apparatur nahe der Siedetemperatur des Acetonitrils mit Chlor umgesetzt. Die Wärmezufuhr zum Verdampfungsgefäss wird dabei so geregelt, dass die vom Kondensator (i) in die Vertiefung (c) zurücklaufende Menge Acetonitril etwa 400 g/Std. (etwa 9,8 Mol/Std.) beträgt. Nach dem Beginn des Rücklaufs des Acetonitrils wird Chlorgas in einer Menge von etwa 35 g/Std. (etwa 0,49 Mol/Std.) durch den Einlass (d) in die Vertiefung (c) geleitet. Das Reaktionsgemisch fliesst durch das Loch in der Vertiefung (c) in das Rohr (e). Dabei sind der Acetonitrilrücklauf und der Chlorstrom so zu regeln, dass der Anteil an Monochloracetonitril im Rohr (e) nicht 33 Mol-% übersteigt und am Ende des Kühlers praktisch kein Chlorgas entweicht. Bei 89%igem Umsatz wird die Chlorierung abgebrochen. Das Produktgemisch im Verdampfungskolben hat laut gaschromatographischer Analyse folgende Zusammensetzung:

86,23% Monochloracetonitril
11,12% Acetonitril
1,96% Trichloracetonitril
0,69% Dichloracetronitril

Nach fraktionierter Destillation erhält man 290 g des 99,5%igen Monochloracetonitrils vom Kp. = 125 bis 125 °C (n$_D^{20}$ = 1.422).

**Patentansprüche**

Verfahren zur Herstellung von reinem Monochloracetonitril durch direkte Chlorierung von Acetonitril in flüssiger Phase unter Einwirkung von UV-Strahlung und gegegebenenfalls in Gegenwart eines Halogenids des 3. bis 6. Gruppe des Periodensystems, dadurch gekennzeichnet, dass man die Chlorierung bei Siedetemperatur des Acetonitrils kontinuierlich so weit durchführt, dass die Konzentration des entstehenden Monochloracetonitrils in dem ständig aus dem Reaktionsraum abgezogenen Reaktionsgemisch nicht mehr als etwa 33 Mol-%, bezogen auf die Molzahl aller in diesem Reaktionsgemisch enthaltenen Stoffe, beträgt.

**Revendications**

Procédé pour la préparation de monochloracétonitrile pur par chloration directe de l'acétonirile en phase liquide avec action de radiations UV et éventuellement en présence d'halogénures des 3e–6e groupes de la Classification Périodique, caractérisé en ce que l'on effectue la chloration en continu jusqu'à ce que la concentration du monochloracétonitrile formé dans le mélange de réaction soutiré en continu de la zone de réaction ne dépasse pas environ 33 moles % par rapport au nombre molaire total de toutes les substances contenues dans le mélange de réaction.

**Claims**

Process for the preparation of pure monochloroacetonitrile by the direct chlorination of acetonitrile in the liquid phase under the action of UV radiation and optionally in the presence of a halide of group 3 to 6 of the periodic table, characterised in that the chlorination is carried out continuously at the boiling point of the acetonitrile to an extent such that the concentration of the resulting monochloroacetonitrile in the reaction mixture constantly withdrawn from the reaction space is not more than about 33 mol %, based on the number of mols of all of the substances contained in this reaction mixture.

FIG.1